# EUROPEAN PATENT APPLICATION

(11) **EP 4 302 803 A2**
(43) Date of publication of application: **10.01.2024**
(21) Application number: 23211704.4
(22) Date of filing: 26.08.2021
(51) Int. Cl.: A61M 5/142

(54) **SYSTEMS AND METHODS FOR ACTIVATING DRUG DELIVERY DEVICES**

(30) Priority: 27.08.2020 US 202063071196 P
(62) Divisional of application: 21790596.7
(71) Applicant: Insulet Corporation, Acton MA 01720 (US)
(72) Inventor: NAZZARO, David, Groveland, MA (US); O'CONNOR, Jason, Acton, MA (US); MCLAUGHLIN, Ian, Groton, MA (US); LEE, Joon Bok, Acton, MA (US); METZMAKER, Thomas, Harvard, MA (US); CHRISTENSEN, Bret, Lexington, MA (US); HILLDALE, Philip, Raleigh, NC (US)
(74) Representative: Peterreins Schley

(57) **Abstract**

Embodiments of the present disclosure relate to approaches for transitioning a drug delivery device from a low-energy sleep state to a high-energy active state. In some embodiments, a system comprising a drug delivery device, wherein the drug delivery device comprises a pump mechanism coupled to a reservoir for expelling a liquid drug from the reservoir, and an activation component communicatively coupled to the pump mechanism. The activation component comprises a location device and a wake-up circuit, wherein the wake-up circuit is operable to transition the drug delivery device from a low-energy state to an active state in response to a detected proximity of the location device to a predetermined location or to a predetermined object.

## Description

### TECHNICAL FIELD

The disclosed examples generally relate to medication delivery. More particularly, the disclosed examples relate to techniques, processes, devices or systems for activating drug delivery devices without the use of an external interface device.

### BACKGROUND

Wearable drug delivery devices are integrated devices, which combine a fluid reservoir, a pumping mechanism, and a mechanism for inserting an integrated subcutaneous cannula. The wearable drug delivery device is adhesively attached to an infusion site on the patient's skin, and typically does not require the use of a separate infusion or tubing set. Some wearable devices deliver a liquid drug (e.g., insulin) to the patient over a period of time via the cannula. The wearable drug delivery device may wirelessly communicate with a separate controller device, such as a personal diabetes manager (PDM).

Activating or "waking-up" the wearable drug delivery device is important since the wearable drug delivery device is typically stored in a low-energy state (e.g., based on µA or nA current draw) after manufacturing. The low current draw is usually provided to maintain memory, clock, etc., as well as other components. Additionally, the low-energy state allows for other peripheral devices to discover the wearable drug delivery device to pair for control and/or share data. However, challenges remain around passively or actively waking up wearable drug delivery devices so the devices are ready to operate fully, especially when peripheral devices like a PDM, phone, or other smart devices are not present.

Accordingly, there is a need for simplified systems and methods for activating drug delivery devices.

### SUMMARY

The present invention is directed to a system as defined in claim 1 and to a method as defined in claim 10. This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

The system of the present invention comprises a drug delivery device, wherein the drug delivery device comprises a pump mechanism coupled to a reservoir for expelling a liquid drug from the reservoir, and an activation component communicatively coupled to the pump mechanism, the activation component comprising a location device and a wake-up circuit. According to the present invention, the wake-up circuit is operable to transition the drug delivery device from a low-energy state to an active state in response to a detected proximity of the location device to a predetermined location or to a predetermined object.

In one approach, the activation component may also include at least one of a sensor and a mechanical activation device, and a wake-up circuit. The wake-up circuit may be operable to receive an input from the sensor or the mechanical activation device, wherein the input indicates a change in a device characteristic, and based on the input from the sensor or the mechanical activation device, transition the drug delivery device from a low-energy state to an active state.

In another approach, a method may include providing a drug delivery device including an activation component comprising a wake-up circuit and at least one of a sensor and a mechanical activation device, and receiving, at the wake-up circuit, an input from the sensor or the mechanical activation device, wherein the input indicates a change in a device characteristic of the drug delivery device. The method may further include transitioning the drug delivery device from a low-energy state to an active state based on the input from the sensor or the mechanical activation device.

In another approach, a non-transitory computer readable medium is embodied with programming code executable by a processor, and the processor, when executing the programming code, may be operable to perform functions, including functions to receive, at a wake-up circuit, an input from a sensor or a mechanical activation device of a drug delivery device, wherein the input indicates a change in a device characteristic of the drug delivery device. The processor when executing the programming code may be operable to perform functions, including functions to transition, in response to the input from the sensor or the mechanical activation device, the drug delivery device from a low-energy state to an active state by activating a controller of the drug delivery device in response to activation of a power supply.

### BRIEF DESCRIPTION OF THE DRAWINGS

In the drawings, like reference characters generally refer to the same parts throughout the different views. In the following description, various embodiments of the present disclosure are described with reference to the following drawings, in which:
**FIG. 1** illustrates an example of a system according to embodiments of the present disclosure;
**FIG. 2** illustrates an example of an activation component of the drug delivery system of **FIG. 1** according to embodiments of the present disclosure; and
**FIG. 3** illustrates a process flow according to embodiments of the present disclosure.

The drawings are not necessarily to scale. The drawings are merely representations, not intended to portray specific parameters of the disclosure. The drawings are intended to depict exemplary embodiments of the disclosure, and therefore are not considered as limiting in scope. Furthermore, certain elements in some of the figures may be omitted, or illustrated not-to-scale, for illustrative clarity. Still furthermore, for clarity, some reference numbers may be omitted in certain drawings.

### DETAILED DESCRIPTION

Systems, devices, and methods in accordance with the present disclosure will now be described more fully with reference to the accompanying drawings, where one or more embodiments are shown. The systems, devices, and methods may be embodied in many different forms and are not to be construed as being limited to the embodiments set forth herein. Instead, these embodiments are provided so the disclosure will be thorough and complete, and will fully convey the scope of the systems, devices, and methods to those skilled in the art. Each of the systems, devices, and methods disclosed herein provides one or more advantages over conventional systems, devices, and methods.

Embodiments of the present disclosure are directed to a drug delivery system and associated methods of operation to change a drug delivery device from a low-energy sleep state to a high-energy active state. The change in device state may be triggered by one or more device characteristic changes determined, for example, through the use of one or more sensors or a mechanical activation device. By using a triggering event to "wake up" the onboard electronics (e.g., a wake-up circuit) from the low-energy state to an active state, (e.g., removal of a sterile barrier of the drug delivery device or contact between the drug delivery device and the user's skin), the onboard electronics of the drug delivery device can provide significantly reduced power consumption after manufacture and during storage and distribution.

In some embodiments, the mechanical activation device may be a tab, cord, or other electrically insulating feature, which is inserted into the wake-up circuit of the onboard electronics so that removal of the mechanical activation device connects the battery or other power supply, thus awakening the electronics from the low-energy state to the active state to perform the required functions. Alternatively, removal of the barrier may cause a switch to close, completing a circuit with the power supply, and thus powering up the system from the low-energy state to the active state.

Furthermore, embodiments of the present disclosure may use the determined energy state change to control operation of the drug delivery device. For example, the system may include a controller that is coupled to the sensor and/or the mechanical activation device, as well as one or more additional components of the drug delivery device. The controller may be adapted structurally or programmed (if electrical or electro-mechanical) to activate or to inhibit these components of the drug delivery device once activated.

Still furthermore, embodiments of the present disclosure may communicate the determined energy state change to another device or system, which may then control operation of the drug delivery device. For example, the system may communicate the energy state change with a networked device using a communication link. In this sense, a networked device is intended to include any device that communicates with at least one other device over a communication link, and might include communication with a device such as a mobile device (e.g., a cell phone or mobile computing device) using a Bluetooth connection or a computing device using a Wi-Fi connection, for example. The networked device may communicate the energy state change to other computing devices remote from the drug delivery device over the network that includes the networked device such as a server.

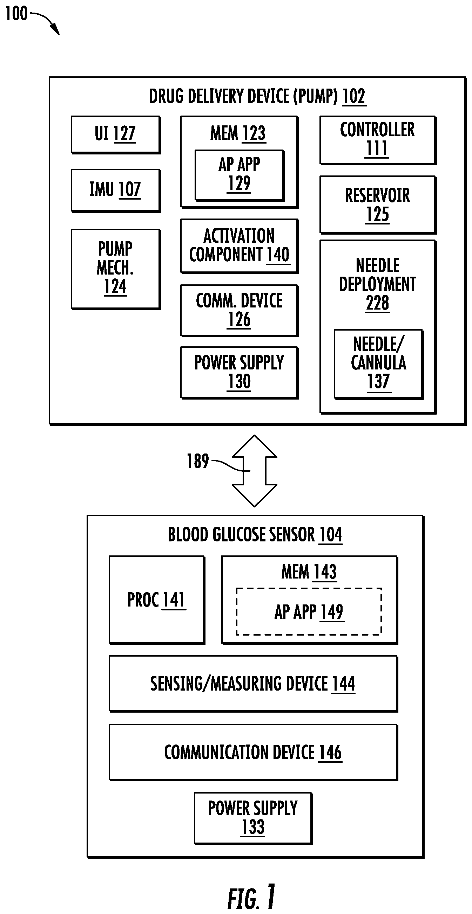

**FIG. 1** illustrates an example of a drug delivery system 100. Various examples of the drug delivery system 100 include a wearable drug delivery device 102 that may operate to manage treatment of a diabetic user according to a diabetes treatment plan. The diabetes treatment plan may include a number of parameters related to the delivery of insulin that may be determined and modified without the use of an external management device.

As shown, the drug delivery system 100 may include a blood glucose sensor 104 communicably coupled to the drug delivery device 102. The drug delivery device 102 may include an inertial measurement unit (IMU) 107, a pump mechanism 124, and a needle deployment component 128. In various examples, the pump mechanism 124 may include a pump or a plunger (not shown). The needle deployment component 128 may include a needle/cannula 137, and any other fluid path components for coupling the stored liquid drug in a reservoir 125 to the user. The cannula 137 may form a portion of the fluid path component coupling the user to the reservoir 125. After the needle deployment component 128 has been activated, a fluid path to the user is provided, and the pump mechanism 124 may expel the liquid drug from the reservoir 125 to deliver the liquid drug to the user.

The wearable drug delivery device 102 may further include a controller 121 and a communications interface device 126. The controller 121 may be implemented in hardware, software, or any combination thereof. The controller 121 may, for example, be a processor, microprocessor, a logic circuit, or a microcontroller coupled to a memory. The controller 121 may maintain a date and time as well as other functions (e.g., calculations or the like) performed by processors. The controller 121 may be operable to execute an artificial pancreas algorithm (AP app) 129 stored in memory 123 that enables the controller 121 to direct operation of the drug delivery device 102. In addition, the controller 121 may be operable to receive data or information indicative of the activity of the user from the IMU 107, as well as from any other sensor, such the blood glucose sensor 104. As will be described in greater detail below, the controller 121 may be further operable to receive data from one or more sensors of an activation component 140 for activating the drug delivery device 102 from a low energy state to an active state.

The controller 121 may process the data from the IMU 107 or any other coupled sensor to determine if an alert or other communication is to be issued to the user and/or a caregiver of the user or if an operational mode of the drug delivery device 102 is to be adjusted. The controller 121 may provide the alert, for example, through the communications interface device 126. The communication link provided by the communications interface device 126 may include any wired or wireless communication link operating according to any known communications protocol or standard, such as RFID, Bluetooth, NFC, or a cellular standard.

In some embodiments, the blood glucose sensor 104 may be, for example, a continuous glucose monitor (CGM). The blood glucose sensor 104 may be physically separate from the drug delivery device 102 or may be an integrated component within a same housing. The blood glucose sensor 104 may provide the controller 121 with data indicative of measured or detected blood glucose levels of the user.

The drug delivery system 100 may be operable to implement the AP application 129, which includes functionality to provide insulin therapy to users without the need for additional user actions beyond filling of insulin in the pump and placing the system onto the user's body. The AP application 129 may further include functionality to define insulin therapy settings, wake the drug delivery device 102 from a dormant state to an active state, and control other functions of the drug delivery device 102, such as needle insertion. The drug delivery system 100 may be an automated drug delivery system capable of activating the drug delivery device 102 without reliance upon a PDM.

The wearable drug delivery device 102 may frequently be referred to as a pump, or an insulin pump, in reference to the operation of expelling a drug from the reservoir 125 for delivery of the drug to the user. In an example, the wearable drug delivery device 102 may include the reservoir 125 for storing the liquid drug, such as insulin or morphine, or other therapeutic drug, the needle/cannula 137 for delivering the drug into the body of the user (which may be done subcutaneously, intraperitoneally, or intravenously), and the pump mechanism 124, or other drive mechanism, for transferring the drug from the reservoir 125, through the needle/cannula 137, and into the user. The pump mechanism 124 may be fluidly coupled to the reservoir 125, and communicatively coupled to the controller 121. The wearable drug delivery device 102 may also include a power supply 130, such as a battery, a piezoelectric device, or the like, for supplying electrical power to the pump mechanism 124 and/or other components (such as the controller 121, memory 123, and the communication interface device 126) of the wearable drug delivery device 102. In some embodiments, the blood glucose sensor 104 may also include a power supply 133. As will be described in greater detail below, the power supply 130 and/or the power supply 133 may be further operable to receive data from one or more sensors of an activation component 140 for activating the drug delivery device 102 from a low energy state to an active state.

In some embodiments, the IMU 107 may be operable to detect various motion parameters (e.g., acceleration, deceleration, speed, orientation, such as roll, pitch, yaw, compass direction, or the like) that may be indicative of the activity of the user. For example, the IMU 107 may output signals in response to detecting motion of the wearable drug delivery device 102 that is indicative of a status of any physical condition of the user, such as, for example, a motion or position of the user. Based on the detected activity of the user, the drug delivery device 102 may adjust operation related to drug delivery and/or device activation.

In some embodiments, the wearable drug delivery device 102 may, when operating in a normal mode of operation, provide insulin stored in reservoir 125 to the user based on information (e.g., blood glucose measurement values, target blood glucose values, insulin on board, prior insulin deliveries, time of day, day of week, inputs from the IMU 107, global positioning system-enabled devices, Wi-Fi-enabled devices, or the like) provided by the blood glucose sensor 104 or other functional elements on drug delivery device 102. For example, the wearable drug delivery device 102 may contain analog and/or digital circuitry that may be implemented as the controller 121 for controlling delivery of the drug or therapeutic agent. The circuitry used to implement the controller 121 may include discrete, specialized logic and/or components, an application-specific integrated circuit, a microcontroller or processor that executes software instructions, firmware, programming instructions or programming code enabling, for example, the AP App 129 stored in memory 123, or any combination thereof. For example, the controller 121 may execute a control algorithm, such as the AP application 129, and other programming code, that may make the controller 121 operable to cause the pump to deliver doses of the drug or therapeutic agent to the user at predetermined intervals or as needed to bring blood glucose measurement values to a target blood glucose value. Furthermore, the size and/or timing of the doses may be pre-programmed, for example, into the AP application 129 by the user or by a third party (such as a health care provider, a parent or guardian, a manufacturer of the wearable drug delivery device, or the like) using a wired or wireless link.

In some embodiments, the sensor 104 may include a processor 141, a memory 143, a sensing or measuring device 144, and a communication device 146. The memory 143 may store an instance of an AP application 149 as well as other programming code and be operable to store data related to the AP application 149.

Instructions for determining the delivery of the drug or therapeutic agent (e.g., as an adjustable basal or bolus dosage) to the user (e.g., the size and/or timing of any doses of the drug or therapeutic agent) may originate locally by the drug delivery device 102 or may originate remotely and be provided to the wearable drug delivery device 102. In an example of a local determination of drug or therapeutic agent delivery, programming instructions, such as an instance of the AP application 129, may be used to make determinations by the wearable drug delivery device 102. In addition, the wearable drug delivery device 102 and the blood glucose sensor 104 may communicate via one or more communication links 189.

In various embodiments, the sensing/measuring device 144 may include one or more additional sensing elements, such as a blood glucose measurement element, a heart rate monitor, a blood oxygen sensor element, or the like. The processor 141 may include discrete, specialized logic and/or components, an application-specific integrated circuit, a microcontroller or processor that executes software instructions, firmware, programming instructions stored in memory (such as memory 143), or any combination thereof.

The wearable drug delivery device 102 may also include a user interface 127, which may include any mechanism for the user to input data to the drug delivery device 102, such as, for example, a button, a knob, a dial, a switch, a touch-screen display, or any other user interaction component. The user interface 127 may include any mechanism for the drug delivery device 102 to relay data to the user and may include, for example, a numbered dial or knob, a display, a touch-screen display, or any means for providing a visual, audible, or tactile (e.g., vibrational) output (e.g., as an alert). The user interface 127 may also include a number of additional components not specifically shown for the sake brevity and explanation. For example, the user interface 127 may include one or more user input/output components for receiving inputs from or providing outputs to a user or a caregiver (e.g., a parent or nurse), a display that outputs a visible alert, a speaker that outputs an audible alert, or a vibration device that outputs tactile indicators to alert a user or a caregiver of a potential activity or operational mode, a power level, and the like. Inputs to the user interface 127 may, for example, be a via a fingerprint sensor, a tactile input sensor, a button, a touch screen display, a switch, or the like. In yet another alternative, changes to the operation of the drug delivery device 102 may be requested through a management device (not shown) that is communicatively coupled to the controller 121.

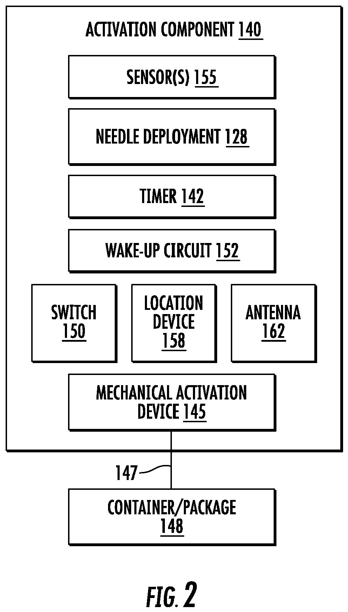

Turning now to **FIG. 2****,** operation of the activation component 140 according to embodiments of the present disclosure will be described in greater detail. In some embodiments, the activation component 140 is operable with the controller 121 and the power supply 130 to trigger the drug delivery device 102 from a dormant or sleep state to an active state, for example, without the use of a PDM. The drug delivery device 102 may arrive to the user or HCP in a low-energy state (e.g., based on µA or nA current draw) after manufacturing. The low-current draw is usually provided to maintain memory and a timer 142, as well as other components of the drug delivery device 102 and/or blood glucose sensor 104.

As shown, the activation component 140 may include a wake-up circuit 152 operable to receive an input corresponding to a device characteristic change. For energy saving, the wake-up circuit 152 works to start the controller 121 preferably only when a change is detected in, for example, a sensor 155 or a mechanical activation device 145. The wake-up circuit 152 may contain analog and/or digital circuitry implemented for controlling activation of the drug delivery device 102. In some embodiments, the wake-up circuit 152 may include discrete, specialized logic and/or components, an application-specific integrated circuit, a microcontroller or processor that executes software instructions, firmware, programming instructions or programming code. The wake-up circuit 152 is operable to receive an input or a signal from the sensor 155, the mechanical activation device 145, and/or other component of the activation component 140, and to activate the controller 121 by initially causing the power supply 130 to turn on. In some embodiments, the wake-up circuit 152 is a subcomponent of the controller 121.

In one example, movement and/or a change in condition of the mechanical activation device 145 of the activation component 140 may provide an input to the wake-up circuit 152, signaling the start of device activation. For example, a portion of a container or package 148 housing the drug delivery device 102 may be directly physically attached to the mechanical activation device 145, which may include a connection component 147 (e.g., pull tab, cord, threading, tie, etc.). Although non-limiting, the package 148 may be a bulk multi-device box (e.g., a 10-pack carton) or a blister pack. In the case the connection component 147 is secured to a flap or cover of the package 148, opening of the flap/cover will cause the connection component 147 to be pulled or removed from the drug delivery device 102. In one non-limiting embodiment, pulling or removing the connection component 147 from the drug delivery device 102 may change a state of a switch 150, e.g., from open to closed or from closed to open. The state change of the switch 150 may be communicated to the wake-up circuit 152, indicating a desire to activate the drug delivery device 102. In one example, the switch 150 may be a proximity switch (e.g., light, magnetic, capacitive) or a reed switch, wherein removal of the drug delivery device 102 from a package of multiple drug delivery devices activates the switch. In some embodiments, the switch forms a part of the wake-up circuit 152.

In another example, the switch 150 may be connected to the fluid reservoir 125 of the drug delivery device 102. During filling of the fluid reservoir 125, touching a metal syringe component to a metal component of the fluid reservoir 125, or elsewhere within the drug delivery device 102, may cause the circuit of the switch 150 to be closed. Additionally, a needle may deform or break the circuit of the switch 150 during filling of the fluid reservoir 125. The change in open/closed state of the switch 150 may be recognized by the wake-up circuit 152 to activate the drug delivery device 102.

In another example, the mechanical activation device 145 may be a disposable portion of the drug delivery device 102, such as a needle cap which, upon removal, electrically opens or closes a circuit, or changes magnetic properties thereof to signal a disconnection. Alternatively, the mechanical activation device 145 may include one or more adhesive release liners, which may be capacitive, resistive, conductive, etc. In yet another example, in the case the needle deployment component 128 is separate/removable from the drug delivery device 102, the presence or removal of the needle deployment component 128 may send a signal to the wake-up circuit 152 to activate the drug delivery device 102.

In another example, the mechanical activation device 145 may be an on-body interlock, such as a button, lever, etc. During operation, opening of the package 148 causes the interlock to passively move to an "off-body" position. After the reservoir 125 is filled, and the drug delivery device 102 is attached to the body of the user, the interlock goes to an "on-body" position, which automatically triggers the timer 142 to start a timing cycle. The timing cycle may act as a buffer or delay to ensure no further movements are made. At the end of the timing cycle, the needle deployment component 128 may be activated, and insulin delivered through the needle/cannula 137. Alternatively, the user must perform an affirmative physical interaction (e.g., push a button, pull a tab, slide a translating member, tap/twist/squeeze a portion of the drug delivery device, etc.) to start the timer 142. In one embodiment, a change of the interlock to the "off-body" position signals the wake-up circuit 152 to activate the drug delivery device 102. In another embodiment, filling of the reservoir 125 to a predetermined fill level signals the wake-up circuit 152 to activate the drug delivery device 102.

In some embodiments, detection of one or more device/user characteristics by the sensor 155 of the activation component 140 may trigger initiation of device activation. Although shown as part of the activation component 140, it will be appreciated that the sensor 155 may be housed within the blood glucose sensor 104 or located separate from the drug delivery device 102 and the blood glucose sensor 104. In one example, the sensor 155 may be an ambient light sensor operable to detect a light change when, e.g., the package 148 is initially opened. The sensor 155 may send a signal to the wake-up circuit 152 to activate the drug delivery device 102 to allow other input devices to measure presence, for example, of an on-body interlock. In some cases, if the drug delivery device 102 does not see activity on one or more other passive actuators/sensors and the package 148 is recognized by the sensor 155 as being dark again, the drug delivery device 102 may be transitioned back into the low-energy state from the active state. In another example, the initial opening of the package 148 may allow one or more drug delivery devices 102 within the package 148 to send data to a cloud network that the package 148 is in the patient or HCP's hands, and that the drug delivery devices are likely to be used in relatively short order, e.g., within days, weeks or months.

In another example, the sensor 155 may be a conductive or capacitance sensor, including associated electronics, operable to measure capacitance and interpret a capacitance value as positive contact with the user's skin. The presence of a body part in contact with, or in close proximity to, the sensor 155, causes a change in a bulk capacitance observed by the sensor 155. More specifically, in some embodiments, the sensor 155 may include an electrified plate sensing surface. The wake-up circuit 152 may include driver electronics coupled to the sensor 155, wherein the driver electronics can include a driver integrated circuit. The driver electronics may continually change a voltage applied to the electrified plate sensing surface. An amount of current required to change a voltage is measured by the driver integrated circuit and indicates an amount of capacitance between the sensor 155 and the skin. Alternatively, the sensor 155 may be a printed circuit board with a plurality of traces. A capacitance digital converter can be included that converts capacitive input signals into a digital value. The digital value can be stored at an on-chip register for interpretation by the wake-up circuit 152. In response to the change in capacitance detected by the sensor 155, the wake-up circuit 152 may activate the drug delivery device 102.

In another example, the sensor 155 may be a temperature and/or humidity sensor. During use, as the sensor 155 is secured to the skin of the user, a change in temperature and/or humidity will be detected. It will be appreciated that the temperature sensor may include any mechanism for measuring temperature and communicating temperature information to the wake-up circuit 152. For example, the temperature sensor may detect skin temperature, acoustic volume sensing (AVS) temperature, ambient temperature, fluid temperatures, among others. In response to the change in temperature and/or humidity detected by the sensor 155, the wake-up circuit 152 may activate the drug delivery device 102.

In another example, the sensor 155 may be a single or multi-axis accelerometer. During use, the drug delivery device 102 can be shaken for a specific time duration (e.g., 5 seconds) and/or with a predetermined shake pattern or sequence (e.g., shake five times in a row). Alternatively, the drug delivery device 102 can be tapped by a finger or hand of the user a specific number of times that correspond to a predetermined number, pattern, or sequence. For example, the user taps drug delivery device 102 two, three, four, five, six, seven, eight, nine, or ten times, and the sensor detects such tapping and activates the drug delivery device once the predetermined number of taps have occurred. In response to the change in acceleration detected by the sensor 155, the wake-up circuit 152 may activate the drug delivery device 102. Sensor 155 and wake-up circuit 152 can be programmed to detect a particular pattern of taps, such as the familiar "shave and a haircut" or "two bits" rhythmic pattern involving seven taps or knocks on a housing or on a particular location of the drug delivery device 102, and upon such detection, activate the drug delivery device 102. Activation of drug delivery device 102 may comprise an alert to the user that the drug delivery device 102 has indeed been activated. Such an alert may comprise one or more beeps or light flashes, thereby notifying the user that drug delivery device 102 has been activated. The alert may correspond to the shaking or tapping pattern or sequence that the user used to activate the device. For example, the alert that is emitted upon activation may comprise the same rhythmic pattern that the user used to activate the device, such as seven beeps in the same "two bits" pattern of taps performed by the user to activate the device.

In another example, the sensor 155 may be a voice-recognition device. Although non-limiting, the voice-recognition device may include a microphone housed in the drug delivery device 102 or the blood glucose sensor 104 or the packaging containing a number of drug delivery devices 102. In response to the sensor 155 receiving a voice input, the wake-up circuit 152 can interpret the statement to activate the drug delivery device 102. In one embodiment, a serial number or other device identifying data may be received by the sensor 155 and recognized by the wake-up circuit 152 to activate the drug delivery device 102.

In another example, drug delivery device 102 may be activated upon receipt of a Bluetooth or other network signal from a third-party voice assistant, such as Amazon's Alexa, Google Assistant, or Microsoft's Cortana. Drug delivery device 102 may be placed within proximity of the third party voice assistant, and upon the user asking the voice assistant to activate the drug delivery device 102 generally (e.g., "Hey Google, activate my Omnipod"), or with a specific code (e.g., "Alexa, activate my Omnipod number 6350972"), the third party voice assistant may send a Bluetooth or other network signal from the third party voice assistant to the drug delivery device 102. Such signal may include the specific code that is linked to the particular drug delivery device 102 that the user is attempting to activate, which specific code may be found on packaging of the drug delivery device 102 or on the device 102 itself for reference by the user. Upon receipt of the Bluetooth or other network signal, drug delivery device 102 may be activated. Upon activation, drug delivery device 102 may send a response signal via Bluetooth or another protocol, to the third party voice assistant, and upon receipt, the third party voice assistant may announce that the drug delivery device 102 has been activated (e.g., "Your Omnipod number 6350972 has been activated").

In another example, the sensor 155 may be a threshold-based sound detection sensor/microphone, which is configured as a one-time trigger tuned to detect the sound resulting from one instance of a specific event. Although non-limiting, the specific audible event may be the opening of a vacuum sealed package, which then signals the wake-up circuit 152 to begin the activation sequence of the drug delivery device 102.

In another example, the sensor 155 may be a pressure sensor, which is operable to generate pressure data in response to pressure conditions within or around the drug delivery device 102. In one embodiment, the package 148 may be vacuum sealed, wherein pressure within the package 148 is drawn to a set value. Upon opening the package 148, the change in pressure will be detected by the sensor 155 and interpreted by the wake-up circuit 152 to activate the drug delivery device 102.

In another example, the sensor 155 may be a gas detector, which is operable to generate gas data in response to gas conditions within or around the drug delivery device 102. In one embodiment, the package 148 may be filled with inert gas (e.g., argon), which has the benefit of slowing the aging process of some components of the drug delivery device 102. During use, opening of the package 148 will cause the sensor 155 to be exposed to standard atmosphere. The change in gas composition will be detected by the sensor 155 and recognized by the wake-up circuit 152 to activate the drug delivery device 102.

In another example, the sensor 155 may be a strain gauge or strain sensor, which recognizes a change in strain as the drug delivery device 102 is removed from packaging or as the drug delivery device 102 is attached to the user, for example. Although non-limiting, the strain gauge may be a foil or wire embedded within or affixed to an adhesive layer of drug delivery device 102. Positive or negative pressure applied to the strain gauge will tensilely or compressively strain the wire, altering its electrical resistivity. The resulting altered electrical resistivity measurements detected by the sensor 155 may be recognized by the wake-up circuit 152 to activate the drug delivery device 102.

In another example, the activation component 140 may further include a location device 158, such as a radio frequency identification (RFID) device, a near-field communication (NFC) device, GPS device, low-power digital radio, and/or a WiFi device. Based on a detected proximity to a predetermined location (e.g., HCP facility, room in house, etc.) or proximity to a predetermined object (e.g., insulin vial, fill station, fill hood, smart phone, etc.) the wake-up circuit 152 may cause the drug delivery device 102 to activate.

In another example, the activation component 140 may further include an antenna 162, such as an RFID antenna. The antenna 162 may be located within the cannula 137 or within the fluid reservoir 125, for example. A transmitter/receiver of the communications interface device 126 may periodically beacon the antenna 162, receiving a signal back so long as the antenna 162 remains out of contact with a fluid (e.g., insulin within the cannula 137 or fluid reservoir 125). Once the antenna 162 is wetted however, it stops transmitting a signal, or the signal is altered in a detectable way. The signal change from the antenna 162 may be interpreted by the wake-up circuit 152 to mean the fluid reservoir 125 has been filled and the drug delivery device 102 may begin activation. In some embodiments, one or more additional antennas 162 may be provided, outside the fluid path, to periodically provide a signal to indicate that there are no faults with the transmit/receive hardware of the communications interface device 126.

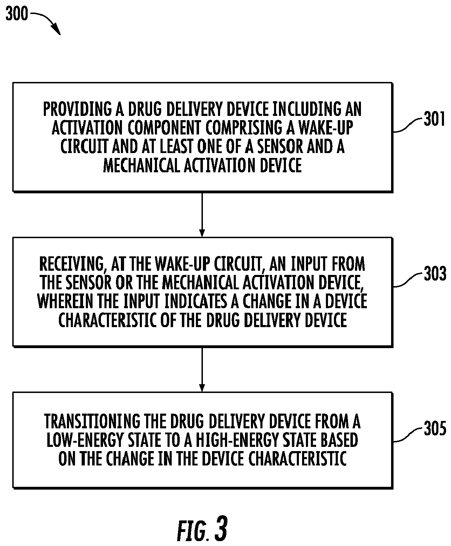

**FIG. 3** illustrates an example process 300 implemented by the system 100 and the activation component 140 according to embodiments of the present disclosure. At block 301, the process 300 may include providing a drug delivery device including an activation component comprising a wake-up circuit and at least one of a sensor and a mechanical activation device. In some embodiments, the drug delivery device may be communicatively coupled to a blood glucose sensor. In some embodiments, the sensor includes at least one of the following: an accelerometer operable to detect a change in acceleration of the drug delivery device, a light sensor operable to detect a change in ambient light conditions surrounding the drug delivery device, a capacitive sensor operable to detect a capacitive input from a user's skin, a temperature sensor operable to detect a change in temperature surrounding the drug delivery device, a humidity sensor operable to detect a change in humidity surrounding the drug delivery device, a voice-recognition sensor operable to detect an audio input, a pressure sensor operable to detect a change in pressure surrounding the drug delivery device, a gas detector operable to detect a change in gas composition surrounding the drug delivery device, and a strain sensor coupled to the drug delivery device, the strain sensor operable to detect a change in tensile or compressive strain. In some embodiments, the mechanical activation device may include a connection component directly coupled to a container, wherein a configuration change of the container causes a corresponding configuration change of the connection component.

At block 303, the process 300 may include receiving, at the wake-up circuit, an input from the sensor or the mechanical activation device, wherein the input indicates a change in a device characteristic of the drug delivery device.

At block 305, the process 300 may include transitioning the drug delivery device from a low-energy state to an active state based on the change in the device characteristic. In some embodiments, transitioning the drug delivery device from the low-energy state to the active state may include activating a power supply of the drug delivery device, and activating a controller of the drug delivery device in response to the activation of the power supply. In some embodiments, transitioning the drug delivery device from the low-energy state to the active state may include detecting a proximity of a location device of the drug delivery device to a predetermined location or to a predetermined object. In some embodiments, the drug delivery device may transition from the low-energy state to the active state without communication between the drug delivery device and an external interface device.

Any of the drug delivery systems, devices, and/or pumps disclosed herein, can be an OmniPod^{®} (Insulet Corporation, Acton, Mass.) insulin delivery device and/or can be any of the drug delivery systems, devices, and/or pumps described in U.S. Provisional Pat. Application Serial Nos. 63/072,417 and 63/150,871, which are incorporated herein by reference in their entirety and for all purposes.

The techniques described herein for a drug delivery system (e.g., the system 100 or any components thereof) may be implemented in hardware, software, or any combination thereof. Any component as described herein may be implemented in hardware, software, or any combination thereof. For example, the system 100 or any components thereof may be implemented in hardware, software, or any combination thereof. Software related implementations of the techniques described herein may include, but are not limited to, firmware, application specific software, or any other type of computer readable instructions that may be executed by one or more processors. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

Some examples of the disclosed devices may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosed subject matter were described above. It is, however, expressly noted that the present disclosed subject matter is not limited to those examples, but rather the intention is that additions and modifications to what was expressly described herein are also included within the scope of the disclosed subject matter. Moreover, it is to be understood that the features of the various examples described herein were not mutually exclusive and may exist in various combinations and permutations, even if such combinations or permutations were not made express herein, without departing from the spirit and scope of the disclosed subject matter. In fact, variations, modifications, and other implementations of what was described herein will occur to those of ordinary skill in the art without departing from the spirit and the scope of the disclosed subject matter. As such, the disclosed subject matter is not to be defined only by the preceding illustrative description.

Program aspects of the technology may be thought of as "products" or "articles of manufacture" typically in the form of executable code and/or associated data that is carried on or embodied in a type of machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example.

The foregoing description of example examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

In addition, although the present invention has been described above and is defined in the claims, it should be understood that the present invention can alternatively be defined in accordance with the following embodiments:
1. A system, comprising: a drug delivery device, comprising: a pump mechanism coupled to a reservoir for expelling a liquid drug from the reservoir; and an activation component communicatively coupled to the pump mechanism, the activation component, comprising: at least one of a sensor and a mechanical activation device; and a wake-up circuit operable to: receive an input from the sensor or the mechanical activation device, wherein the input indicates a change in a device characteristic; and based on the input from the sensor of the mechanical activation device, transition the drug delivery device from a low-energy state to an active state.
2. The system of embodiment 1, the drug delivery device further comprising a power supply communicatively coupled to the wake-up circuit, wherein the wake-up circuit is further operable to transition the drug delivery device from the low-energy state to the active state by activating the power supply.
3. The system of embodiment 1 or 2, the drug delivery device further comprising a controller communicatively coupled to the power supply, wherein activation of the power supply causes activation of the controller.
4. The system of one of the preceding embodiments, further comprising a blood glucose sensor communicatively coupled to the drug delivery device.
5. The system of one of the preceding embodiments, wherein the sensor includes at least one of the following: an accelerometer operable to detect a change in acceleration of the drug delivery device, a light sensor operable to detect a change in ambient light conditions surrounding the drug delivery device, a capacitive sensor operable to detect a change in an amount of capacitance from a user's skin, a temperature sensor operable to detect a change in temperature surrounding the drug delivery device, a humidity sensor operable to detect a change in humidity surrounding the drug delivery device, a voice-recognition sensor operable to detect an audio input, a pressure sensor operable to detect a change in pressure surrounding the drug delivery device, a gas detector operable to detect a change in gas composition surrounding the drug delivery device, and a strain sensor coupled to the drug delivery device, the strain sensor operable to detect a change in tensile or compressive strain.
6. The system of one of the preceding embodiments, wherein the mechanical activation device comprises a connection component directly coupled to a container housing the drug delivery device, and wherein a configuration change of the container causes a corresponding configuration change of the connection component.
7. The system of one of the preceding embodiments, wherein the mechanical activation device is a detachable needle cap, and wherein the wake-up circuit is further operable to transition the drug delivery device from the low-energy state to the active state in response to the detachable needle cap being removed.
8. The system of one of the preceding embodiments, wherein the activation component further comprises a location device, and wherein the wake-up circuit is further operable to transition the drug delivery device from the low-energy state to the active state in response to a detected proximity of the location device to a predetermined location or to a predetermined object.
9. The system of one of the preceding embodiments, wherein the activation component further comprises a switch communicatively coupled to the wake-up circuit, wherein the wake-up circuit is further operable to transition the drug delivery device from the low-energy state to the active state in response to a state change of the switch.
10. A method, comprising: providing a drug delivery device including an activation component comprising a wake-up circuit and at least one of a sensor and a mechanical activation device; receiving, at the wake-up circuit, an input from the sensor or the mechanical activation device, wherein the input indicates a change in a device characteristic of the drug delivery device; and transitioning the drug delivery device from a low-energy state to an active state based on the input from the sensor or the mechanical activation device.
11. The method of embodiment 10, wherein transitioning the drug delivery device from the low-energy state to the active state comprises: activating a power supply of the drug delivery device; and activating a controller of the drug delivery device in response to the activation of the power supply.
12. The method of embodiment 10 or 11, further comprising directly coupling a connection component of the mechanical activation device to a container, wherein the drug delivery device is housed by the container, and wherein a configuration change of the container causes a corresponding configuration change of the connection component.
13. The method of one of embodiments 10 to 12, wherein the input received from the sensor includes at least one of the following: a change in acceleration of the drug delivery device, a change in ambient light conditions surrounding the drug delivery device, a change in an amount of capacitance from a user's skin, a change in temperature surrounding the drug delivery device, a change in humidity surrounding the drug delivery device, an audio input, a change in pressure surrounding the drug delivery device, a change in gas composition surrounding the drug delivery device, and a strain sensor coupled to the drug delivery device, a change in tensile or compressive strain.
14. The method of one of embodiments 10 to 13, wherein transitioning the drug delivery device from the low-energy state to the active state comprises detecting a proximity of a location device of the drug delivery device to a predetermined location or to a predetermined object.
15. The method of one of embodiments 10 to 14, further comprising causing the drug delivery device to transition from the low-energy state to the active state without communication between the drug delivery device and an external interface device.
16. A non-transitory computer readable medium embodied with programming code executable by a processor, and the processor when executing the programming code is operable to perform functions, including functions to: receive, at a wake-up circuit, an input from a sensor or a mechanical activation device of a drug delivery device, wherein the input indicates a change in a device characteristic of the drug delivery device; and transition, in response to the input from the sensor or the mechanical activation device, the drug delivery device from a low-energy state to an active state by activating a controller of the drug delivery device in response to activation of a power supply.
17. The non-transitory computer readable medium of embodiment 16, further embodied with programming code executable by the processor, and the processor when executing the programming code is operable to performing functions to: detect a change in acceleration of the drug delivery device, detect a change in ambient light conditions surrounding the drug delivery device, detect a change in an amount of capacitance from a user's skin, detect a change in temperature surrounding the drug delivery device, detect a change in humidity surrounding the drug delivery device, an audio input, detect a change in pressure surrounding the drug delivery device, a change in gas composition surrounding the drug delivery device, detect a strain sensor coupled to the drug delivery device, or detect change in tensile or compressive strain.
18. The non-transitory computer readable medium of embodiment 17, further embodied with programming code executable by the processor, and the processor when executing the programming code is operable to transition the drug delivery device from the low-energy state to the active state by detecting a proximity of a location device of the drug delivery device to a predetermined location or to a predetermined object.
19. The non-transitory computer readable medium of embodiment 17 or 18, further embodied with programming code executable by the processor, and the processor when executing the programming code is operable to transition the drug delivery device from the low-energy state to the active state in response to a configuration change of a connection component of the mechanical activation device.
20. The non-transitory computer readable medium of one of embodiments 17 to 19, further embodied with programming code executable by the processor, and the processor when executing the programming code is operable to cause the drug delivery device to transition from the low-energy state to the active state without communication between the drug delivery device and an external interface device.

## Claims

1. A system, comprising:
a drug delivery device (102), comprising:
a pump mechanism (124) coupled to a reservoir (125) for expelling a liquid drug from the reservoir; and
an activation component (140) communicatively coupled to the pump mechanism, the activation component (140) comprising a location device (158) and a wake-up circuit (152),
wherein the wake-up circuit is operable to transition the drug delivery device from a low-energy state to an active state in response to a detected proximity of the location device to a predetermined location or to a predetermined object.

2. The system of claim 1, the drug delivery device further comprising a power supply (130) communicatively coupled to the wake-up circuit, wherein the wake-up circuit is further operable to transition the drug delivery device from the low-energy state to the active state by activating the power supply.

3. The system of claim 2, the drug delivery device further comprising a controller (121) communicatively coupled to the power supply, wherein activation of the power supply causes activation of the controller.

4. The system of one of the preceding claims, further comprising a blood glucose sensor (104) communicatively coupled to the drug delivery device.

5. The system of one of the preceding claims, wherein the activation component further comprises at least one sensor (155), wherein the wake-up circuit is further operable to receive an input from the at least one sensor, wherein the input indicates a change in a device characteristic, and based on the input from the at least one sensor, transition the drug delivery device from the low-energy state to the active state.

6. The system of claim 5, wherein the at least one sensor includes at least one of the following:
an accelerometer operable to detect a change in acceleration of the drug delivery device,
a light sensor operable to detect a change in ambient light conditions surrounding the drug delivery device,
a capacitive sensor operable to detect a change in an amount of capacitance from a user's skin,
a temperature sensor operable to detect a change in temperature surrounding the drug delivery device,
a humidity sensor operable to detect a change in humidity surrounding the drug delivery device,
a voice-recognition sensor operable to detect an audio input,
a pressure sensor operable to detect a change in pressure surrounding the drug delivery device,
a gas detector operable to detect a change in gas composition surrounding the drug delivery device, and/or
a strain sensor coupled to the drug delivery device, the strain sensor operable to detect a change in tensile or compressive strain.

7. The system of one of the preceding claims, wherein the activation component further comprises a mechanical activation device (145), wherein the wake-up circuit is further operable to receive an input from the mechanical activation device, wherein the input indicates a change in a device characteristic, and based on the input from the mechanical activation device, transition the drug delivery device from the low-energy state to the active state.

8. The system of claim 7, wherein the mechanical activation device comprises a connection component directly coupled to a container housing the drug delivery device, and wherein a configuration change of the container causes a corresponding configuration change of the connection component.

9. The system of claim 7, wherein the mechanical activation device is a detachable needle cap, and wherein the wake-up circuit is further operable to transition the drug delivery device from the low-energy state to the active state in response to the detachable needle cap being removed.

10. The system of one of claims 7 to 9, wherein the activation component further comprises a switch communicatively coupled to the wake-up circuit, wherein the wake-up circuit is further operable to transition the drug delivery device from the low-energy state to the active state in response to a state change of the switch.

11. A method, comprising:
providing a drug delivery device (120) including an activation component (140) and a location device (158);
receiving, at a wake-up circuit (152), an input from the activation component (140);
transitioning the drug delivery device (120) from a low-energy state to an active state based on the input from the activation component (140),
wherein transitioning the drug delivery device (120) from the low-energy state to the active state comprises detecting a proximity of the location device (158) of the drug delivery device (120) to a predetermined location or to a predetermined object.

12. The method of claim 11, wherein transitioning the drug delivery device from the low-energy state to the active state comprises:
activating a power supply of the drug delivery device; and
activating a controller (121) of the drug delivery device in response to the activation of the power supply.

13. The method of claim 11 or 12, wherein the activation component comprises a mechanical activation device (145), the method further comprising:
receiving, at the wake-up circuit, an input from the mechanical activation device, wherein the input indicates a change in a device characteristic of the drug delivery device; and
transitioning the drug delivery device from the low-energy state to the active state based on the input from the mechanical activation device.

14. The method of claim 13, further comprising directly coupling a connection component of the mechanical activation device to a container, wherein the drug delivery device is housed by the container, and wherein a configuration change of the container causes a corresponding configuration change of the connection component.

15. The method of one of claims 11 to 14, wherein the activation component comprises at least one sensor (155), the method further comprising:
receiving, at the wake-up circuit, an input from the sensor, wherein the input indicates a change in a device characteristic of the drug delivery device; and
transitioning the drug delivery device from the low-energy state to the active state based on the input from the sensor.

16. The method of claim 15, wherein the input received from the sensor includes at least one of the following:
a change in acceleration of the drug delivery device,
a change in ambient light conditions surrounding the drug delivery device,
a change in an amount of capacitance from a user's skin,
a change in temperature surrounding the drug delivery device,
a change in humidity surrounding the drug delivery device,
an audio input,
a change in pressure surrounding the drug delivery device,
a change in gas composition surrounding the drug delivery device, and/or
a change in tensile or compressive strain.
